(19) Europäisches Patentamt
European Patent Office
Office européen des brevets

(11) Publication number: **0 107 430**
**B1**

(12) **EUROPEAN PATENT SPECIFICATION**

(45) Date of publication of patent specification: **13.08.86**

(21) Application number: **83306096.5**

(22) Date of filing: **07.10.83**

(51) Int. Cl.$^4$: **C 07 C 45/50,** C 07 C 47/02, C 07 C 29/16, C 07 C 31/125, B 01 J 23/46

(54) **Method for the preparation of alcohols and aldehydes by reacting olefins with carbon monoxide and hydrogen.**

(30) Priority: **21.10.82 US 435812**
**21.10.82 US 435813**
**21.10.82 US 435814**

(43) Date of publication of application:
**02.05.84 Bulletin 84/18**

(45) Publication of the grant of the patent:
**13.08.86 Bulletin 86/33**

(84) Designated Contracting States:
**BE DE FR GB NL**

(56) References cited:
**WO-A-80/01692**
**US-A-3 511 880**
**US-A-3 832 391**

(73) Proprietor: **TEXACO DEVELOPMENT CORPORATION**
**2000 Westchester Avenue**
**White Plains New York 10650 (US)**

(72) Inventor: **Knifton, John Frederick**
**10900 Catskill Trail**
**Austin Texas 78750 (US)**
Inventor: **Lin, Jiang-Jen**
**2617 Oak Meadow**
**Round Rock Texas 78664 (US)**
Inventor: **Grigsby, Robert Allison, Jr.**
**216 Deepwood**
**Georgetown Texas 78626 (US)**
Inventor: **Brader, Walter Howe, Jr.**
**8803 Crestridge Circle**
**Austin Texas 78759 (US)**

(74) Representative: **Burnside, Michael et al**
**Michael Burnside & Partners 2 Serjeants' Inn**
**Fleet Street**
**London EC4Y 1HL (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 107 430 B1**

**Description**

This invention concerns an improved process for preparing alcohols and aldehydes by the reaction of terminal or internal olefins with carbon monoxide and hydrogen (otherwise known as synthesis gas or syngas) in the presence of a ruthenium-containing catalyst system.

The processes of hydroformylation and carbonylation are well known in the art and involve reactions represented by:

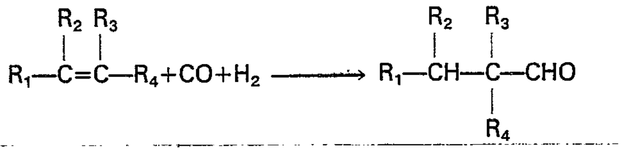

and/or

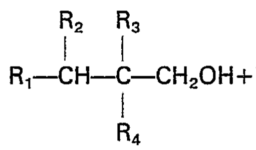 +isomeric alcohols and aldehydes,

wherein $R_1$, $R_2$, $R_3$ and $R_4$ represent a wide variety of monovalent groups. The aldehydes and alcohols produced generally correspond to the compounds obtained by the addition of a carbonyl or carbinol group to an olefinically unsaturated carbon atom, with simultaneous saturation of the olefin bond. Isomerization of the olefin bond may take place to varying degrees under certain conditions, with consequent variation in the products obtained.

The hydroformylation reaction does not generally proceed in the absence of catalysts, and a disadvantage of many of the previously-known hydroformylation processes is their dependence upon the use of catalysts, particularly the commonly-used cobalt-derived homogeneous 'oxo' catalysts, which generally need exceedingly high pressures to remain stable under the other conditions employed. A further disadvantage of many of the known processes is their inability to produce hydroformylation products comprising substantial amounts of alcohols, thereby necessitating a separate aldehyde hydrogenation step when alcohols are a desired product. The production of hydroformylation products having a relatively high normal to branched product isomer ratio is also often exceedingly difficult, if at all possible, in many of the practical scale processes now in use. Another problem in many hydroformylation processes is the formation of by-products in competing reactions. Examples of such unwanted by-products include alkanes, formed by competing olefin hydrogenation, olefin isomers formed by double bond isomerization, ketone formation, and aldols generated as a result of condensation reactions of the product aldehyde.

In commercially-practiced hydroformylation processes, cobalt- and rhodium-catalyzed systems are most commonly used, and while cobalt and rhodium have been the focus of much of the prior hydroformylation research, numerous other metals have been disclosed as catalysts for this synthesis. For a review of the prior art pertaining to the use of cobalt and rhodium-based hydroformylation processes see: R. L. Pruett, "Advances in Organometallic Chemistry", Vol. 17, p. 1 (1979).

Typical of the prior art relating to the use of ruthenium as a hydroformylation catalyst are the publications of Wilkinson et al. In GB 1,138,601, Example 6, the hydroformylation of alpha-olefins (1-hexene) to aldehydes is described using soluble, phosphine-stabilized ruthenium catalyst precursors, such as $[(Ph_2EtP)_6Ru_2Cl_2]Cl$. Moderately high pressures are used, and the use of a two-step technique of hydroformylation and subsequent hydrogenation as a synthetic route to alcohols is discussed. Additional information regarding the use of a variety of tertiary-phosphine-ruthenium complexes in the catalytic hydroformylation of alkenes to aldehydes, particularly the dependence of conversion and aldehyde ratios upon catalyst concentration, temperature, partial and total pressures, nature of the substrate, and the addition of excess phosphine, may be found in a second publication by this group in J. Chem. Soc. p. 399 (1976). Similar classes of catalysts are disclosed also in U.S.A. 3,239,566, which employs a catalyst consisting of a ruthenium or rhodium component in complex combination with carbon monoxide and a trialkylphosphine. Here, the greatest percentage of the converted olefins form alcohols and aldehydes with less than seven carbon atoms.

A variety of multidentate phosphone-platinum complexes useful in catalytic hydroformylation of alkenes to aldehydes are described in U.S.A. 4,229,381.

The use of ruthenium salts, such as ruthenium (III) chloride and ruthenium stearate, as well as ruthenium carbonyls and ruthenium on carbon, as catalyst precursors for the hydroformylation of olefins to straight-chain and branched aldehydes, is disclosed in GB 966,461 and 999,461. U.S.A. 4,306,084 describes an oxo reaction where the ruthenium carbonyl catalyst is maintained in basic solution. Recently the cluster anion, $[HRu_3(CO)_{11}]^-$, has been shown to catalyze the hydroformylation of ethylene and propylene to $C_3$—$C_4$ aldehydes in dimethylformamide at 100°C, see C. Suss-Fink, J. Organomet. Chem., 193, C20 (1980).

Polymer-bound ruthenium hydroformylation catalysts, prepared, for example, by reacting diphenylphosphinated styrene divinylbenzene resins with phosphine-stabilized ruthenium carbonyls, have also been described recently. Pittman, in J. Org. Chem. *46*, 1901 (1981), finds improved normal/branched aldehyde ratios with these resins compared with homogeneous catalyst versions. The more desirable alcohol products are not reported to be formed with this class of ruthenium catalyst.

U.S.A. 3,239,569 discloses the production of aldehydes and alcohols in a single stage conversion which comprises contacting an olefinic hydrocarbon with carbon monoxide and hydrogen in the presence of a catalyst system comprising cobalt in complex combination with carbon monoxide and a trialkylphosphine. Here again, the majority of the hydroformylation products had six carbon atoms or less.

There is therefore a need in the art for a one-stage process of preparing alcohols and aldehydes from olefinically unsaturated compounds, particularly internal olefin compounds, by a process which utilizes lower pressures and results in a high yield of aliphatic alcohols of the $C_8$—$C_{20}$ range, e.g. linear surfactant-grade alcohols.

An object of this invention, therefore, is the oxonation of olefins, particularly higher molecular weight olefin fractions, e.g. $C_7$—$C_{14}$, $C_8$—$C_{15}$ or $C_8$—$C_{20}$ internal olefin fractions, to produce predominantly aliphatic alcohols at pressures lower than previously used and finally, to outline a method of recovering the product alcohol from non-volatile ruthenium catalyst.

According to a preferred embodiment the present invention provides a process for preparing alcohols and aldehydes by reacting $C_2$—$C_{30}$ terminal or internal olefins with carbon monoxide and hydrogen in the presence of a ruthenium catalyst at a temperature of at least 50°C and a pressure of at least 7.5 Bars characterized in that the ruthenium catalyst is dispersed in a low melting quaternary phosphonium or ammonium base or salt, or that the ruthenium catalyst, in association with a tertiary amine or phosphine promoter, is dispersed in a low melting phosphonium base or salt.

Preferred reaction conditions include a temperature from 100 to 220°C, and a pressure from 35 to 210 Bars.

According to one embodiment, the ruthenium catalyst is dispersed in a low melting quaternary phosphonium or ammonium base or salt.

According to another embodiment, the ruthenium catalyst and a monodentate or multidentate tertiary phosphine promoter are dispersed in a low melting quaternary phosphonium base or salt.

According to a further embodiment, the ruthenium catalyst and a monodentate or multidentate tertiary amine promoter are dispersed in a low melting quaternary phosphonium base or salt.

In order to present the inventive concept in the greatest possible detail to promote its understanding, the following supplementary disclosure is submitted. The basic invention, improved upon here, is practiced as follows:

Catalysts that are suitable in the practice of this invention contain ruthenium. The ruthenium-containing catalyst may be chosen from a wide variety of organic or inorganic compounds and complexes, as will be shown and illustrated below. It is only necessary that the catalyst precursor actually employed should contain the metal in any of its ionic states. The actual catalytically active species is then believed to comprise ruthenium in complex combination with carbon monoxide, hydrogen and olefin substrates. One of the most effective catalysts is believed to be achieved where ruthenium hydrocarbonyl species are solubilized in the quaternary salt under reaction conditions.

Other effective catalysts include a ruthenium hydrocarbonyl species in conjunction with an N-heterocyclic amine promoter solubilized in a quaternary phosphonium salt, and a ruthenium species in conjunction with a multidentate phosphine promoter solubilized in a quaternary phosphonium salt.

The ruthenium catalyst precursors may take many different forms. For instance, the ruthenium may be added to the reaction mixture as an oxide, for example, ruthenium(IV) oxide hydrate, anhydrous ruthenium(IV) dioxide or ruthenium(VIII) tetraoxide. Alternatively, it may be added as the salt of a mineral acid, e.g. ruthenium(III) chloride hydrate, ruthenium(III) bromide, ruthenium(III) triiodide, tricarbonyl ruthenium(II) iodide, anhydrous ruthenium(III) chloride or ruthenium nitrate; or as the salt of a suitable organic carboxylic acid, for example, ruthenium(III) acetate, ruthenium naphthenate, or ruthenium valerate. Ruthenium(III) acetylacetonate is also a suitable catalyst precursor. The ruthenium may furthermore be added to the reaction zone as a carbonyl or hydrocarbonyl derivative, e.g. triruthenium dodecacarbonyl and other hydrocarbonyls such as $H_2Ru_4(CO)_{13}$ and $H_4Ru_4(CO)_{12}$, and substituted carbonyl species such as the tricarbonylruthenium(II) chloride dimer $[Ru(CO)_3Cl_2]_2$.

Preferred ruthenium compounds include oxides of ruthenium, ruthenium salts of a mineral acid, ruthenium salts of an organic carboxylic acid and ruthenium carbonyl or hydrocarbonyl derivatives. Among the particularly preferred compounds are ruthenium(IV) dioxide hydrate, ruthenium(III) acetylacetonate, and triruthenium dodecacarbonyl. The usefulness of these ruthenium precursors for alcohol and aldehyde synthesis is illustrated by the accompanying Examples.

The ruthenium-containing compound is, before use, dispersed in a low melting quaternary phosphonium or ammonium base or salt.

The quaternary phosphonium or ammonium base or salt should be relatively low melting, that is, melt at a temperature less than the temperature of reaction necessary for making said alcohols and aldehydes. Usually the quaternary compound has a melting point less than 180°C, and most often has a melting point less than 150°C.

The addition of the quaternary phosphonium or ammonium base or salt to the ruthenium-containing compounds ensures the following improvements in olefin hydroformylation performance:

1) Improved yields of desired product (alcohol plus aldehyde).
2) Less formation of by-product hydrocarbons.
3) Ease of separation of the ruthenium catalyst from the alcohol and aldehyde products.
4) Maintained activity for the ruthenium catalyst during multiple recycling experiments.

Suitable quaternary phosphonium salts for the practice of this invention have the formula:

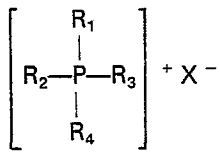

where $R_1$, $R_2$, $R_3$ and $R_4$ are organic radicals, particularly alkyl, aryl or alkaryl radicals bonded to the phosphorus atom, and $X^-$ is an anion. Particularly useful organic radicals include alkyl radicals having 1 to 20 carbon atoms in a branched or linear alkyl chain, e.g. methyl, ethyl, *n*-butyl, *iso*-butyl, octyl, 2-ethylhexyl and dodecyl. Tetraethylphosphonium bromide and tetrabutylphosphonium bromide are typical commercially-available examples. The corresponding quaternary phosphonium acetates, hydroxides, nitrates, chromates, tetrafluoroborates and other halides, such as chlorides and iodides, are also satisfactory. Corresponding quaternary ammonium bases and salts are also useful.

Equally useful are phosphonium and ammonium salts in which phosphorus or nitrogen is bonded to a mixture of alkyl, aryl and alkaryl radicals. These aryl and alkaryl radicals may have 6 to 20 carbon atoms. The aryl radical is most commonly phenyl. The alkaryl group may comprise phenyl substituted by one or more $C_1$ to $C_{10}$ alkyl groups.

Examples of suitable quaternary phosphonium and ammonium bases and salts include tetrabutyl phosphonium bromide, heptyltriphenylphosphonium bromide, tetrabutylphosphonium iodide, tetrabutylphosphonium chloride, tetrabutylphosphonium nitrate, tetrabutylphosphonium hydroxide, tetrabutylphosphonium chromate, tetrabutylphosphonium tetrafluoroborate, tetrabutylphosphonium acetate, tetrabutylammonium bromide, tetraheptylammonium chloride, tetramethylammonium fluoride, tetramethylammonium hydroxide, pentahydrate, n-dodecyltriphenylphosphonium bromide, hexadecyltri-n-butylphosphonium bromide, n-dodecyltributylphosphonium bromide, ethyltriphenylphosphonium iodide, benzyl trimethylammonium hydroxide and trimethyldodecylammonium bromide. Tables 1 and 2 provide evidence of the effectiveness of these quaternary ammonium and phosphonium salts and bases when used in combination with ruthenium(IV) oxide, hydrate, triruthenium dodecacarbonyl and ruthenium(III) acetyl acetonate.

Also suitable in the practice of this invention are N-heterocyclic salts, particularly N-heterocyclic halides such as N-ethylquinolinium iodide and N-methylquinolinium bromide.

The preferred quaternary salts are generally tetraalkylphosphonium salts containing alkyl groups having 1 to 16 carbon atoms, such as methyl, ethyl, butyl, dodecyl and hexadecyl. Preferred tetrabutylphosphonium salts or bases include the bromide, chloride, iodide, acetate and chromate salts and hydroxide base. Tetrabutylphosphonium salts, such as tetrabutylphosphonium bromide, are most preferred.

In one embodiment of the invention, amine promoters are added to the ruthenium catalyst for the purpose of improving the linearity of the product alcohol and aldehyde, i.e., the ratio of straight-chain aliphatic alcohol product to branched chain alcohol product, illustrated here by structures (A) and (B).

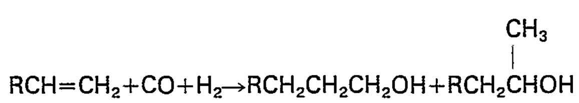

*A* *B*

Many different amine promoters are suitable for use in conjunction with a ruthenium compound and a quaternary phosphonium base or salt in the desired hydroformylation reaction. Generally these promoters contain one or more tertiary substituted nitrogen atoms per molecule, each trisubstituted nitrogen being bonded to one or more carbon atoms. These amines may be aryl or aliphatic amines, or they may be N-heterocyclic amines; they may be diamines, containing two tertiary nitrogen donor atoms per molecule, polyamines or monoamines. The tertiary substituted nitrogen atoms in the diamine and polyamine structures may be separated by 0 to 12 carbon atoms, or they may be bonded to different cyclopentadienyl or arene groups of a metallocene.

Suitable tertiary amine promoters containing one or more N-heterocyclic rings include 2,2′-dipyridyl; pyridine; 1,10-phenanthroline; 3,5 lutidine; 2,6 lutidine; 2,2′-dipyridylamine; 2,3′-dipyridyl; 2,4′-dipyridyl; 2,2′,2″-terpyridyl; 2,4,6 - tri(2 - pyridyl) - s - triazine; 4,4′-dipyridyl; 4,4′-dimethyl-2,2′-dipyridyl, 2,6-diphenylpyridine; diphenyl-2-pyridylmethane; 2,5-dimethylpyrazine; 2,6-dimethoxypyridine; 4-dimethylaminopyridine and isoquinoline.

Suitable aliphatic and aryl tertiary amine promoters include N,N,N',N'-tetramethylethylenediamine; trimethylamine; triethylamine; tri-n-butylamine; N,N'-dimethylpiperazine; N,N,N',N'-tetramethyl-*o*-phenylenediamine; 1,8 - bis - (dimethylamino) - naphthalene; 1,2 - dimethylpyrroline; 1,2-dipiperidinoethane; 1,4-diazabicyclo (2.2.2) octane; N,N,N',N' - tetramethyl - 1,3 - propylenediamine; n-methylpyrrole; and hexamethylene tetramine.

The preferred tertiary amine promoters for the practice of this invention are bidentate N-heterocyclic compounds, such as 2,2'-dipyridyl.

The tertiary amine promoter, added in conjunction with the ruthenium-containing compound is dispersed in the low-melting quaternary phosphonium salt before its catalytic use in making alcohols and aldehydes. It may be noted from the comparative Examples 67 to 69 and 70 to 73 that the addition of the tertiary amine promoter, e.g. 2,2'-dipyridyl, has the effect of:

(i) Increasing the linearity of the product alcohols (e.g. from 65% to 87% in Examples 70 and 72), and

(ii) Lowering the amount of by-product alkane formed (c.f. Examples 72 and 70 as well as Examples 67 and 69).

Where oxonation is performed on linear alpha olefins in this embodiment of the invention, the corresponding OXO alcohols are prepared in good yield with linearity of the alcohol fraction reaching 93%. This selective oxonation reaction is demonstrated by Example 26 and illustrated by the following equation:

$$RCH{=}CH_2+CO+2H_2 \rightarrow RCH_2CH_2CH_2OH$$

Where oxonation is performed on linear alpha olefins to yield primarily aldehyde products, the linearity of said aldehyde fraction may reach 98% when using the ruthenium-amine promoter catalyst systems of this invention. The selective oxonation reaction is demonstrated in Example 27 and illustrated by the following equation:

$$RCH_2{—}CH_2+CO+H_2 \rightarrow RCH_2CH_2CHO$$

In another embodiment of the invention phosphine promoters may be added (similarly to the amines) to improve the linearity of the product alcohol or aldehyde.

Suitable phosphine promoters contain one or more trisubstituted phosphorus atoms per molecule, each bonded to one or more carbon atoms. These phosphines may be disphosphines, containing two phosphorus atoms per molecule, polyphosphines or monophosphines. The trisubstituted phosphorus atoms in the diphosphine and polyphosphine molecules may be separated by 0 to 12 carbon atoms, or bonded to different cyclopentadienyl or arene groups of a metallocene. Phosphine promoters of the bidentate and multidentate type include, but are not limited to, bis(1,3 - diphenylphosphino)propane; bis(1,2 - diphenylphosphino)ethane; bis(1,4 - diphenylphosphino)butane; bis(1,5 - diphenylphosphino)pentane; bis(1,6 - diphenylphosphino)hexane; bis(1,1 - diphenylphosphino)methane; tris(2 - diphenylphosphinoethyl)phosphine; 1,1,1 - tris(diphenylphosphinomethyl)ethane; 1,1' - bis(diphenylphosphino)ferrocene; and bis(2 - diphenylphosphinoethyl)phenyl phosphine.

Monodentate phosphines such as triphenylphosphine and tributylphosphine may also be used, but are generally not as desirable. Substituted promoters, such as tri(2-cyanoethyl)phosphine, are also effective in the application of this ruthenium-catalyzed hydroformylation reaction.

The olefins employed in the practice of this invention include internal and terminal olefins containing 2 to 30 carbon atoms, and mixtures thereof. Examples of suitable olefins include straight-chain terminal olefins such as propylene, 1-butene, 1-octene, 1-decene, 1-dodecene, 1-tetradecene, and 1-hexadecene. Also suitable are branched-chain, terminal olefins such as 3-methyl-1-pentene, 4-methyl-1-hexene, 3,3-dimethyl-1-butene and 3,4-dimethyl-1-hexene. Linear and branched internal olefins are also suitable substrates for this hydroformylation. Examples include 2-octene, 3-octene, 4-octene, mixed internal octenes, mixed internal decenes, mixed internal dodecenes as well as 2-pentene, 3-hexene, 5-decene, 2-decene, 2-dodecene, and 5-methyl-2-hexene. Cyclic olefins, e.g. cyclohexene, cyclopentene, cycloheptene and their branched derivatives such as 1-methylcyclohexene and 2-ethylcyclopentene are also useful in the practice of this invention.

Particularly preferred are straight-chain terminal olefins such as propylene, 1-butene, 1-octene, 1-decene and 1-dodecene, as well as linear internal olefins such as 2-octene, mixed internal octenes, mixed internal undecenes and mixed internal $C_{13}$—$C_{14}$ olefins, as well as terminal, internal olefin mixtures thereof.

The quantity of ruthenium catalyst (exclusive of quaternary salt) employed in the instant invention is not critical and may vary over a wide range. In general, the process according to the invention is desirably conducted in the presence of a catalytically effective quantity of the active ruthenium species and quaternary phosphonium or ammonium salt or base which gives the desired products in reasonable yields. The reaction proceeds when employing as little as $1 \times 10^{-6}$ weight percent, and even lesser amounts, of ruthenium together with as little as $1 \times 10^{-6}$ weight percent of quaternary phosphonium or ammonium salt or base, basis the total weight of the reaction mixture. The upper concentration is dictated by a variety of factors including catalyst cost, partial pressures of carbon monoxide and hydrogen, and the operating

temperature. A ruthenium catalyst concentration of from $1\times10^{-5}$ to 30 weight percent ruthenium in conjunction with a low melting quaternary phosphonium or ammonium salt or base concentration of from 0.1 to 80 weight percent, based on the total weight of reaction mixture is generally desirable in the practice of this invention. The preferred atomic ratio of ruthenium to low melting quaternary phosphonium or ammonium salt or base is from 0.01:1 to 10:1.

The reaction proceeds when employing as little as $1\times10^{-6}$ weight percent, and even lesser amounts, of ruthenium together with as little as $1\times10^{-6}$ weight percent of the amine promoter and as little as $1\times10^{-6}$ weight percent of quaternary phosphonium salt basis the total weight of the reaction mixture. The upper concentration is dictated as before by a variety of factors including catalyst cost, partial pressures of carbon monoxide and hydrogen, and operating temperature. A ruthenium catalyst concentration of from $1\times10^{-5}$ to 30 weight percent ruthenium, in conjunction with an amine promoter concentration of from $1\times10^{-5}$ to 30 weight percent and a low-melting quaternary phosphonium salt concentration of from 0.1 to 80 weight percent, based on the total weight of reaction mixture is generally desirable in the practice of this embodiment of the invention.

The amounts of phosphine promoter are generally in the same range as the amine promoter. The reaction proceeds when employing as little as $1\times10^{-6}$ weight percent, and even lesser amounts, of ruthenium together with at least $1\times10^{-6}$ weight percent of phosphine promoter and at least $1\times10^{-6}$ weight percent of quaternary phosphonium salt, basis the total weight of the reaction mixture. A ruthenium catalyst concentration of from $1\times10^{-5}$ to 30 weight percent ruthenium in conjunction with a phosphine promoter concentration of $1\times10^{-5}$ to 30 weight percent and a low melting quaternary phosphonium salt concentration of from 0.1 to 80 weight percent, based on the total weight of reaction mixture, is generally desirable in the practice of this invention.

The temperature which can usefully be employed in these syntheses is a variable dependent upon other experimental factors, including the pressure, the concentration and the choice of the particular species of ruthenium catalyst and promoters, among other things. The range of operability is from 50° to 350°C when superatmospheric pressures of syngas are employed. 100 to 220°C represents the preferred temperature range.

Superatmospheric pressures of at least 7.5 Bars lead to substantial yields of alcohols and aldehydes by the process of this invention. A preferred operating range is above 35 Bars, and pressures above 210 Bars also provide useful yields of desired alcohols.

The relative amounts of carbon monoxide and hydrogen which may be initially present in the syngas mixture may be varied over a wide range. In general, the mole ratio of CO-to-$H_2$ is in the range from 20:1 up to 1:20, preferably from 5:1 to 1:5, although ratios outside these ranges may be employed. Particularly in continuous operations, but also in batch experiments, the carbon monoxide-hydrogen gaseous mixtures may also be used in conjunction with up to 50% by volume of one or more other gases. These other gases may include one or more inert gases such as nitrogen, argon and neon, or they may include gases that may or may not undergo reaction under CO hydrogenation conditions, such as carbon dioxide; hydrocarbons such as methane, ethane, and propane; ethers such as dimethyl ether, methyl ethyl ether and diethyl ether; alkanols such as methanol; and acid esters such as methyl acetate.

In all these syntheses, the amount of carbon monoxide and hydrogen present in the reaction mixture should be sufficient at least to satisfy the stoichiometry of the desired oxonation reaction.

The major by-products of these alcohol and aldehyde syntheses are commonly alkanes, isomerized olefins, and aldols, formed both through condensations with the product aldehydes, and in some cases, from subsequent dehydration and reduction of the initially formed aldol.

The aldehyde and alcohol products may readily be separated from the ruthenium-catalyst-containing crude product mixture by conventional means, e.g. by fractional distillation in vacuo. The by-products identified above may also be isolated by conventional means, or they may be recycled with the ruthenium catalysts.

The process according to this invention can be conducted in a batch, semi-continuous or continuous fashion. The catalyst may initially be introduced into the reaction zone batchwise, or it may be continuously or intermittently introduced into such a zone during the course of the synthesis reaction. Operating conditions can be adjusted to optimize the formation of the desired alcohol product, and said material may be recovered by methods well known in the art, such as distillation, fractionation, or extraction. A fraction rich in ruthenium catalyst components may then be recycled to the reaction zone, if desired, and additional products generated.

The products have been identified in this work by one or more of the following analytical procedures, viz., gas-liquid phase chromatograph (glc), infrared (ir), mass spectrometry, nuclear magnetic resonance (nmr) and elemental analyses, or a combination of these techniques. Analyses have, for the most part, been by parts in weight; all temperatures are in degrees Centigrade and all pressures in Bars.

The following Examples are submitted to supply specific and illustrative embodiments of the present invention.

Example 1

Ruthenium(IV) oxide hydrate (1.15 g, 6.0 mmole) was dispersed in tetrabutylphosphonium bromide (20.0 g, 58.9 mmole), diluted with 2-octene (22.4 g, 200 mmole) and transferred in a glass liner, under $N_2$

purge, to an 850 ml capacity pressure reactor equipped with heating and means of agitation. The reactor was sealed, flushed with $CO/H_2$ and pressurized to 83.7 Bars with $CO/H_2$ (1:2). The mixture was heated to 180°C with rocking, held at that tempeature for six hours and then allowed to cool.

Upon reaching ambient temperature, the reactor pressure (55.45 Bars) was noted, a typical gas sample taken, and the excess gas removed. The deep-red liquid product (48.3 g) was analyzed by glc and Karl Fischer titration.

The liquid contained:
- 33.9 wt.% 1-nonanol
- 29.7 wt.% 2-methyloctanol
- 6.1 wt.% 2-ethylheptanol
- 3.8 wt.% 1-nonanal
- 1.6 wt.% branched $C_9$ aldehydes
- 9.1 wt.% n-octane
- 1.9 wt.% octenes
- 0.6 wt.% water.

The gas sample contained:
- 53 wt.% hydrogen
- 26 wt.% carbon monoxide
- 20 wt.% carbon dioxide.

Estimated conversion of octene charge=98%.
Estimated total yield of $C_9$ alcohols plus aldehydes=72 mole%.

Example 2 (Comparative)

This Example shows the poorer performance of the ruthenium catalyst in the absence of a quaternary phosphonium salt.

Example 1 was repeated, except that no tetrabutylphosphonium bromide was employed.

Upon reaching ambient temperature, the reactor pressure (66.5 Bars) was noted, a typical off-gas sample taken, and the excess gas removed. The yellow liquid (32 ml) plus orange solids were weighed (27.5 g total) and analyzed.

A typical liquid sample contained:
- 26.5 wt.% 1-nonanol
- 11.8 wt.% 2-methyloctanol
- 2.3 wt.% 2-ethyl heptanol
- 47.7 wt.% octane
- 0.1 wt.% unreacted octenes.

Estimated conversion of octene charge=98%.
Estimated total yield of $C_9$ alcohols plus aldehydes=37 mole%.

In comparing these results with those of Example 1, it may be noted that:

a) The estimated total yield of nonanol plus nonanal products is higher in Example 1, (yield 72 mole% versus 37 mole% in comparative Example 2).

b) The production of by-product hydrocarbon, in this case octane, is lower in Example 1 (e.g. octane concentration in the product mix is 9.1 wt.% against 47.7 wt.% in Example 2).

In the following Examples 2 to 8, a variety of other internal and alpha olefin substrates, particularly mixed internal octenes, mixed internal undecenes, $C_{13}$—$C_{14}$ internal olefin fractions and 1-decene are employed.

Example 3

A dispersion of ruthenium(IV) oxide (0.57 g, 3.0 mmole) in tetrabutylphosphonium bromide (10.0 g, 29.5 mmole) was diluted with 18.2 g of a mixed $C_{13}$—$C_{14}$ internal olefin fraction (ca. 100 mmole) and reacted with $CO/H_2$ as described in Example 1.

Upon reaching ambient temperature, the reactor pressure (76.8 Bars) was noted, a typical gas sample taken, and the excess gas removed. The liquid product (32.1 g) comprised:
- 16.5 wt.% 1-tetradecanol
- 31.6 wt.% branched $C_{14}$ alcohols
- 10.8 wt.% 1-pentadecanol
- 24.4 wt.% branched $C_{15}$ alcohols
- 6.1 wt.% tridecane
- 6.1 wt.% tetradecane
- 0.1 wt.% unreacted $C_{13}$—$C_{14}$ internal olefins.

Estimated conversion of $C_{13}$—$C_{14}$ internal olefins=98%.
Estimated yield of $C_{14}$—$C_{15}$ alcohols=82 mole%.

Example 4

A dispersion of ruthenium(IV) oxide hydrate (79 mmole) in tetrabutylphosphonium bromide (250 g, 737 mmole) was diluted with $C_{11}$ mixed internal olefin (1540 g, 10 mole) and transferred to a 4 litre capacity pressure reactor equipped with heating and means of agitation. The reactor was sealed, flushed with $CO/H_2$ and pressurized to 83.7 Bars with $CO/H_2$ (1:2). The mixture was heated to 180°C with stirring, held at that temperature for 7 hours and then allowed to cool. During the course of the reaction, the vessel was repressurized several times to 125 Bars with $CO/H_2$ (1:2).

Upon reaching ambient temperature, a typical off-gas sample was taken and the excess gas removed. The dark red liquid product (2041 g) was analyzed by glc.

A typical liquid sample contained:
19.8 wt.% 1-dodecanol
21.1 wt.% 2-methyl undecanol
19.8 wt.% 2-ethyldecanol
0.5 wt.% dodecanal
16.9 wt.% undecane
5.1 wt.% unreacted undecenes.

Typical gas samples contained:
51.8 wt.% hydrogen
14.6 wt.% carbon monoxide
28.2 wt.% carbon dioxide.

Estimated conversion of $C_{11}$ internal olefin charge=92.7%.
Estimated total yield of $C_{12}$ alcohols plus aldehydes=65.9%.

A portion of the liquid product (1694 g) was vacuum distilled. Liquid distillate fractions were recovered as water-white liquids. Their combined weight was 706 g. A typical composition for a distillate fraction having a B.P. range 21—106°C was as follows:
5.4 wt.% 1-dodecanol
11.7 wt.% 2-methyl undecanol
14.7 wt.% 2-ethyldecanol
0.6 wt.% 1-dodecanal
2.8 wt.% branched aldehydes
34.1 wt.% undecane/undecene.

The pot residue (988 g) was recovered as a deep red liquid which contained ruthenium carbonyl species, tetrabutylphosphonium bromide, $C_{12}$ alcohols and some heavier fractions.

Example 5

A mixture of ruthenium(IV) oxide, hydrate (82 mmole), 1-decene (1500 g, 10.71 mole) and tetrabutylphosphonium bromide (250 g, 737 mole) was added to a 4 litre capacity pressure reactor equipped with heating and means of agitation. The reactor was sealed, flushed with $CO/H_2$ and pressurized to 83.7 Bars with $CO/H_2$ (1:2). The mixture was heated to 180°C and agitation was started. At temperature the reactor was connected to a surge tank containing $CO/H_2$ (1:2), and the pressure in the reactor was raised to 152.7 Bars, held at this value at 180°C for 23 hours, and then the reaction mixture was allowed to cool.

Upon reaching ambient temperature, a typical gas sample was taken and the excess gas removed. The deep-red liquid product (2058 g) was analyzed by glc, showing the following composition:
49.9 wt.% 1-undecanol
24.0 wt.% 2-methyldecanol
4.8 wt.% 2-ethylnonanol
12.6 wt.% decane.

Typical gas samples contained:
47.8 wt.% hydrogen
22.3 wt.% carbon monoxide
21.2 wt.% carbon dioxide.

Estimated conversion of 1-decene=100%.
Estimated total yield of $C_{11}$ alcohols plus aldehydes=77.2%.

Example 6

A mixture of ruthenium(IV) oxide, hydrate (1.5 mmole) in tetrabutylphosphonium bromide (5.0 g, 11.7 mmole) was diluted with $C_{11}$ mixed internal olefin (22.8 g, 150 mmole) and 1-tetradecanol (8.5 g, 40.0 mmole) and transferred in a glass liner, under $N_2$ purge, to a 300 ml capacity pressure reactor equipped with heating and agitation means. The reactor was sealed, flushed with $CO/H_2$ and pressurized to 83.7 Bars with $CO/H_2$ (1:2). The mixture was heated to 180°C with rocking, held at this temperature for 4 hours and then allowed to cool.

Upon reaching ambient temperature, the reactor pressure (49.25 Bars) was noted, a typical gas sample taken, and the excess gas removed. The dark brown product is analyzed by glc.

A typical liquid sample contained:

8.8 wt.% 1-dodecanol
9.1 wt.% 2-methylundecanol
7.5 wt.% 2-ethyldecanol
1.2 wt.% 1-dodecanal
10.5 wt.% n-undecane
20.1 wt.% unreacted undecenes
20.7 wt.% 1-tetradecanol.

Typical gas samples contained:

55.7 wt.% hydrogen
31.6 wt.% carbon monoxide
10.9 wt.% carbon dioxide.

Estimated conversion of undecene charge=70.5%.
Estimated yield of $C_{12}$ alcohols plus aldehydes=52.7%.

Example 7

A solution (2821 g) containing the pot residue (35.0%) from Example 4 and a $C_{11}$ mixed internal olefin (65.0%) was pumped into a 1 litre continuous unit until it was filled. The unit was then heated slowly to 180°C under 132 Bars of $CO/H_2$ (1:2). The solution was then pumped into the continuous unit at a rate of 50 g/hr. After 5 hours the unit reached steady-state conditions. Typical gas samples were taken. Samples of the deep red liquid product were collected and analyzed by glc.

Analysis of a typical liquid sample, once the reactor reached steady-state conditions, was as follows:

12.5 wt.% 1-dodecanol
12.3 wt.% 2-methylundecanol
10.3 wt.% 2-ethyldecanol
4.3 wt.% 1-dodecanal
9.2 wt.% branched $C_{12}$ aldehydes
7.9 wt.% undecane
23.6 wt.% unreacted undecenes.

Typical gas samples contained:

62.0 wt.% hydrogen
29.8 wt.% carbon monoxide
5.8 wt.% carbon dioxide.

Estimated conversion of undecene charge=76.3%.
Estimated total yield of $C_{12}$ alcohols plus aldehydes=63.9%.

Example 8

A solution containing the pot residue (35.0%) from Example 4 and a $C_{11}$ internal olefin fraction (65.0%) was pumped (75 g/hr) into a fluid, 1 litre continuous unit kept at 220°C under $CO/H_2$ (1:2) pressure of 132 Bars. After 3 hours, the unit reached steady-state conditions. Typical gas samples were taken. Samples of the deep red liquid product were collected and analyzed by glc.

Analysis of a typical liquid sample, once the reactor reaches steady-state conditions, was as follows:

18.6 wt.% 1-dodecanol
25.6 wt.% 2-methylundecanol
24.2 wt.% 2-ethyldecanol
0.1 wt.% dodecanal
0.7 wt.% branched $C_{12}$ aldehydes
14.2 wt.% undecane
3.6 wt.% unreacted undecenes.

Typical gas samples contained:
55.0 wt.% hydrogen
35.4 wt.% carbon monoxide
4.2 wt.% carbon dioxide.

Estimated conversion of octene charge=96.4%.
Estimated total yield of $C_{12}$ alcohols/aldehydes=71.6%.

Table 1 illustrates the effect of employing different quaternary phosphonium salts with different ruthenium sources. Starting with mixed internal octenes, yields of nonanols plus nonanals exceed 80 mole % for the $RuO_2$—$C_{16}H_{33}Bu_3Br$ and $RuO_2$—$Bu_4PI$ combinations (see Example 9 and 13). Linearity of the $C_9$ alcohol product is 69% for the ruthenium(IV) oxide-tetrabutylphosphonium acetate couple (Example 16).

TABLE 1
Hydroformylation of internal octenes

| Example | Ruthenium source | Reaction media[a] | Liquid product composition (%): Octenes | Octane | Nonanals: Branch | Nonanals: Linear | Nonanols: Branched 2-Et | Nonanols: Branched 2-Me | Nonanols: Linear | Octene conv. (%) | Nonanols+ nonanals yield (%) |
|---|---|---|---|---|---|---|---|---|---|---|---|
| 9 | $RuO_2 . xH_2O$ | $C_{16}H_{33}Bu_3PBr$ | 0.1 | 12.2 | 0.2 | 0.3 | 10.6 | 34.5 | 35.0 | 99 | 81 |
| 10 | " | $C_7H_{15}Ph_3PBr$ | 0.1 | 15.0 | 0.3 | 0.2 | 9.9 | 32.6 | 34.2 | 99 | 74 |
| 11 | " | $C_{12}H_{25}Ph_3PBr$ | 0.1 | 13.4 | 0.1 | 0.3 | 9.9 | 32.4 | 33.1 | 99 | 74 |
| 12 | " | $Bu_4PBr$ | 0.2 | 14.2 | 0.3 | 0.2 | 9.8 | 32.9 | 32.1 | 99 | 76 |
| 13 | " | $Bu_4PI$ | 0.4 | 9.8 | 1.2 | 0.1 | 10.9 | 34.4 | 35.3 | 99 | 83 |
| 14 | " | $EtPh_3PI$ | 8.3 | 13.8 | 9.9 | 2.4 | 4.9 | 21.9 | 26.5 | 90 | 68 |
| 15 | " | $Bu_4PCl$ | 0.1 | 10.0 | 0.2 | 0.3 | 10.0 | 33.0 | 34.4 | 99 | 76 |
| 16 | " | $Bu_4POAc$ | 41.4 | 5.9 | 1.9 | 0.3 | 2.1 | 9.1 | 24.3 | 54 | 59 |
| 17 | $Ru_3(CO)_{12}$ | $Bu_4PBr$[b] | 0.1 | 18.2 | 0.4 | 0.4 | 9.4 | 30.3 | 28.9 | 99 | 66 |
| 18 | $Ru(acac)_3$ | " | 0.4 | 14.8 | 0.3 | 0.7 | 10.4 | 30.8 | 27.5 | 99 | 66 |

[a] Typical reaction charge: Ru, 3.0 mmole; quaternary phosphonium salt, 5.0 g; mixed internal octenes, 100 mmole.
Typical run conditions: 180°C, 83.7 Bars $CO/H_2$ (1:2) initial pressure, 4 hours.
[b] Typical reaction charge: Ru, 6.0 mmole, $Bu_4Pbr$, 20.0 g; octenes, 200 mmole.

Table 2 illustrates the case of ruthenium(IV) oxide, hydrate dispersed in different quaternary ammonium salts such as di(octadecyl)dimethylammonium chloride, tetrabutylammonium bromide and tetraheptylammonium chloride (Examples 19 to 21), as well as use of N-heterocyclic salts such as N-ethylquinolinium iodide (Example 25).

TABLE 2
Hydroformylation of internal octenes

| Example | Ruthenium source | Reaction media[a] | Octenes | Octane | Liquid product composition: Nonanals Branch | Nonanals Linear | Nonanols Branched 2-Et | Nonanols Branched 2-Me | Nonanols Linear |
|---|---|---|---|---|---|---|---|---|---|
| 19 | $RuO_xxH_2O$ | $(C_7H_{15})_4NCl$ | 5.4 | 11.0 | 4.0 | 0.7 | 5.1 | 19.4 | 22.5 |
| 20 | " | $Bu_4NBr$ | 11.9 | 0.2 | 0.1 | 0.9 | 6.0 | 23.9 | 27.6 |
| 21 | " | $(C_{18}H_{37})_2Me_2NCl$ | 0.1 | 5.6 | 1.4 | 0.2 | 8.8 | 25.7 | 21.5 |
| 22 | " | $Me_4NF$ | [d] 32.3<br>0.5 | 59.8<br>2.3 | 0.5 | 0.1 | 0.1 | | 0.3 |
| 23 | " | $Me_4NOAc$ | [d] 81.2<br>32.3 | 10.0 | 0.6 | 0.5<br>18.5 | | 0.1 | 0.1 |
| 24 | " | $BzMe_3NOH$[c] | [d] 40.0<br>10.8 | 2.2<br>0.8 | 4.3<br>3.0 | 4.6<br>3.9 | 0.2<br>0.2 | 0.2<br>0.2 | 0.8<br>1.0 |
| 25 | " | Et QUIN . I[b] | [d] 90.8<br>12.3 | 2.4 | 0.3 | 0.4 | | | |

[a] Typical reaction charge: Ru, 3.0 mmole; quaternary ammonium salt, 5.0 g; octenes, 100 mmole.
Typical run conditions: 180°C; 83.7 Bars, $CO/H_2$ (1:2) initial pressure, 4 hours.
[b] Et QUIN . I, N-ethylquinolinium iodide.
[c] Added as 40% solution in methanol.
[d] A two-phase liquid product.

The following Examples 26 to 89 illustrate the use of tertiary amine promoters.

Example 26

Ruthenium(IV) oxide (1.146 g, 6.0 mmole) plus 2,2'-dipyridyl (0.937 g, 6.0 mmole) was dispersed in tetra-n-butylphosphonium bromide (10.0 g, 29.5 mmole), diluted with 1-octene (22.4 g, 200 mmole) and transferred in a glass liner, under $N_2$ purge, to an 850 ml capacity pressure reactor equipped with heating and agitation means. The reactor was sealed, flushed with $CO/H_2$ and pressurized to 138.9 Bars, total pressure, with $CO/H_2$ (1:2). The mixture was heated to 160°C with rocking, held at this temperature for 4 hours and then allowed to cool.

Upon reaching ambient temperature, the reactor pressure (111.3 Bars) was noted, a typical gas sample taken, and the excess gas vented. The red liquid product (39.4 g) was analyzed by glc and Karl Fischer titration. It had the following composition:

17.6 wt.% octene
10.6 wt.% octane
28.2 wt.% linear nonanal
5.9 wt.% branched nonanals
26.6 wt.% linear nonanol
2.1 wt.% branched nonanols
3.0 wt.% water.

Estimated linearity of the nonanol fraction is 93%. Estimated conversion of the octene charge is 79%. Estimated yield of nonanals plus nonanols (basis octene converted) is 76 mole%.

Example 27

The mixture of Example 26 was reacted at 100°C with rocking, for 4 hours and then allowed to cool.

Upon reaching ambient temperature, the reactor pressure (82 Bars) was noted, a typical gas sample taken, and the excess gas vented. The two-phase liquid product (35.3 g) was analyzed by glc and Karl Fischer titration.

The lighter (top phase) liquid product (29 ml) contained:

81.3 wt.% octene
18.4 wt.% linear nonanal
0.2 wt.% branched nonanals
0.1 wt.% linear nonanol
3.0 wt.% water.

Estimated linearity of the nonanal fraction in this product phase is 98.9%.

The brown-colored, heavier (bottom-phase) liquid product (13 ml) contained:

49.1 wt.% octene
1.9 wt.% octane
34.0 wt.% linear nonanals
0.4 wt.% branched nonanals
4.6 wt.% linear nonanols.

Estimated linearity of the nonanal fraction in this phase is 98.8%. Estimated total conversion of the octene charge is 27%. Estimated total yield of nonanals plus nonanols (basis octene charge) is 77 mole%.

Examples 28 to 49

In these Examples, the equipment and procedures of Example 26 were used, the reactor charge in each case being ruthenium(IV) oxide, hydrate (6.0 mmole), 2,2'-dipyridyl (6.0 mmole), tetra-n-butylphosphonium bromide (10 g) and 1-octene (22.4 g, 200 mmole), but different operating temperatures, pressures and $CO/H_2$ mole ratios were used. The data are summarized in Table 3.

It may be noted that:

[a] Aldehyde product linearity reaches 97.6% in Example 28.
[b] Alcohol product linearity reaches 97.6% in Examples 33, 39, 40 and 43.
[c] Total alcohol plus aldehyde yield (basis octene converted) was 85 mole% in Example 28.
[d] A wide range of operating conditions may be employed with the ruthenium-amine catalysts of this invention.

TABLE 3

| Example | Operating temp. °C | Initial pressure (bars) $H_2$ | CO | Total | Liquid yield (g) | Octene | Octane | Product composition % Nonyl aldehydes Linear | Branched | Nonyl alcohols Linear | Branched | $H_2O$ | Alcohol linearity | Aldehyde linearity |
|---|---|---|---|---|---|---|---|---|---|---|---|---|---|---|
| 28 | 120 | 55.6 | 28.1 | 83.7 | 25.9 | 37.3 | 4.5 | 49.1 | 1.2 | 2.8 | | 5.1 | | 97.6 |
| 29 | 140 | " | " | " | 26.2 | 26.4 | 7.7 | 49.8 | 1.9 | 9.4 | 1.4 | 4.7 | 87 | 96 |
| 30 | 160 | " | " | " | 27.1 | 16.7 | 8.6 | 21.8 | 5.1 | 34.2 | 4.0 | 3.2 | 90 | |
| 31 | 180 | " | " | " | 27.3 | 10.4 | 11.5 | 0.5 | 1.5 | 55.3 | 14.2 | 1.4 | 80 | |
| 32 | 200 | " | " | " | 28.1 | 1.0 | 15.5 | 0.1 | 0.5 | 48.8 | 25.9 | 1.1 | 65 | |
| 33 | 180 | 21.2 | 62.55 | " | 25.5 | 10.8 | 7.5 | 11.3 | 4.8 | 42.5 | 4.4 | 0.8 | 91 | |
| 34 | 180 | 28.1 | 55.6 | " | 27.6 | 8.7 | 8.6 | 4.0 | 3.9 | 48.0 | 8.1 | 1.7 | 86 | |
| 35 | 180 | 41.9 | 41.9 | " | 27.6 | 8.8 | 8.8 | 2.1 | 3.2 | 49.7 | 9.3 | 1.2 | 84 | |
| 36 | 180 | 55.65 | 28.1 | " | 27.3 | 10.4 | 11.5 | 0.5 | 1.5 | 55.3 | 14.2 | 1.4 | 80 | |
| 37 | 180 | 62.55 | 21.2 | " | 27.4 | 12.7 | 10.4 | 1.5 | 3.8 | 49.3 | 15.2 | 1.7 | 76 | |
| 38 | 160 | 28.0 | 14.3 | 42.3 | 26.9 | 22.1 | 11.2 | 1.1 | 1.9 | 41.6 | 14.9 | 1.3 | 74 | |
| 39 | 160 | 55.6 | 28.1 | 83.7 | 27.1 | 16.7 | 8.6 | 21.8 | 5.1 | 34.2 | 4.0 | 3.2 | 90 | |
| 40 | 160 | 92.2 | 46.2 | 138.4 | 27.3 | 17.6 | 10.6 | 28.2 | 5.9 | 26.6 | 2.1 | 3.0 | 93 | |
| 41 | 160 | 138.4 | 69.45 | 207.85 | 27.4 | 17.1 | 10.0 | 30.1 | 6.2 | 22.5 | 2.8 | 6.2 | 89 | |
| 42 | 180 | 55.65 | 55.65 | 111.3 | 28.2 | 8.3 | 7.8 | 10.0 | 5.3 | 43.5 | 5.5 | 1.9 | 89 | |
| 43 | 180 | 55.65 | 110.85 | 166.5 | 28.2 | 9.8 | 6.7 | 23.4 | 7.6 | 29.5 | 3.3 | 2.9 | 90 | |
| 44 | 180 | " | 166 | 221.65 | 27.0 | 9.7 | 4.1 | 12.1 | 7.3 | 31.8 | 8.1 | 3.0 | 80 | |
| 45 | 180 | 14.3 | 28.0 | 42.3 | 27.1 | 16.0 | 6.0 | 6.7 | 4.5 | 41.9 | 6.8 | 2.0 | 86 | |
| 46 | 180 | 28.0 | " | 56.0 | 25.6 | 15.8 | 8.7 | 4.1 | 2.7 | 46.5 | 6.5 | 2.1 | 88 | |
| 47 | 180 | 83.25 | " | 111.3 | 27.9 | 5.7 | 6.1 | 3.9 | 4.4 | 57.8 | 12.3 | 2.2 | 82 | |
| 48 | 180 | 110.8 | " | 138.8 | 28.0 | 9.1 | 13.9 | 0.5 | 2.3 | 52.3 | 14.5 | 1.6 | 78 | |
| 49 | 180 | 138.4 | " | 166.4 | 28.3 | 17.4 | 18.5 | 2.0 | 5.1 | 38.8 | 10.9 | 3.2 | 78 | |

Examples 50 to 66

In these Examples, the equipment and procedures of Example 26 were used, the operating temperature (180°C) initial pressure (83.7 Bars $CO/H_2$ (1:2)), and reaction time (4 hours) being the same, but different concentrations and mole ratios of reactants are employed. The data are summarized in Table 4.

It may be noted that a wide range of ruthenium, 2,2′-dipyridyl, and 1-octene concentrations may be employed in the practice of this invention. Linearity of the product nonyl alcohols routinely exceeded 75% except in the absence of the 2,2′-dipyridyl promoter (Example 55).

TABLE 4

| Example | Catalyst precursor | Olefin feed | Liquid yield (g) | Product composition (%) Octene | Octane | Nonyl aldehydes Linear | Nonyl aldehydes Branched | Nonyl alcohols Linear | Nonyl alcohols Branched | $H_2O$ | Alcohol linearity (%) |
|---|---|---|---|---|---|---|---|---|---|---|---|
| 50 | 1/4 ($RuO_2$-BIPY)[a] | 1-Octene | 26.1 | 21.2 | 13.7 | 18.3 | 12.3 | 19.3 | 4.0 | 4.7 | 83 |
| 51 | 1/2 ($RuO_2$-BIPY) | 1-Octene | 26.5 | 14.6 | 7.8 | 20.2 | 13.5 | 24.6 | 7.7 | 1.4 | 76 |
| 52 | $RuO_2$-BIPY | 1-Octene | 26.9 | 15.4 | 7.9 | 14.8 | 10.2 | 31.0 | 9.1 | 1.5 | 77 |
| 53 | 2 ($RuO_2$-BIPY) | 1-Octene | 27.1 | 16.8 | 9.2 | 5.9 | 6.9 | 40.8 | 13.2 | 2.1 | 76 |
| 54 | 3 ($RuO_2$-BIPY) | 1-Octene | 27.7 | 15.5 | 14.1 | 0.4 | 1.3 | 49.5 | 14.2 | 2.1 | 78 |
| 55 | $RuO_2$ | 1-Octene | 27.4 | — | 16.8 | 0.2 | 1.5 | 38.5 | 22.1 | 0.9 | 64 |
| 56 | $RuO_2$-1/4 BIPY | 1-Octene | 27.9 | 9.0 | 13.1 | 4.3 | 10.1 | 31.6 | 10.1 | 3.4 | 76 |
| 57 | $RuO_2$-1/2 BIPY | 1-Octene | 26.2 | 10.0 | 16.9 | 11.3 | 11.2 | 28.9 | 7.5 | 2.3 | 79 |
| 58 | $RuO_2$-BIPY | 1-Octene | 26.9 | 15.4 | 7.9 | 14.8 | 10.2 | 31.0 | 9.1 | 1.5 | 77 |
| 59 | $RuO_2$-1½ BIPY | 1-Octene | 29.9 | 13.8 | 13.9 | 8.1 | 7.9 | 37.7 | 7.0 | 2.0 | 84 |
| 60 | $RuO_2$-2 BIPY | 1-Octene | 26.8 | 15.3 | 12.6 | 7.4 | 7.3 | 40.9 | 9.0 | 1.4 | 82 |
| 61 | $RuO_2$-3 BIPY | 1-Octene | 26.7 | 13.4 | 11.4 | 4.5 | 5.6 | 42.3 | 9.2 | 2.4 | 82 |
| 62 | $RuO_2$-BIPY[b] | ½[1-Octene] | 13.0 | 12.4 | 7.8 | 10.7 | 7.9 | 28.0 | 5.5 | 3.2 | 84 |
| 63 | $RuO_2$-BIPY[b] | 1-Octene | 26.5 | 14.6 | 7.8 | 20.2 | 13.5 | 24.6 | 7.7 | 1.4 | 76 |
| 64 | $RuO_2$-BIPY[b] | 1½[1-Octene] | 39.8 | 18.7 | 13.5 | 17.9 | 13.7 | 20.4 | 3.9 | 2.8 | 81 |
| 65 | $RuO_2$-BIPY[b] | 2[1-Octene] | 52.2 | 22.1 | 13.9 | 21.4 | 15.7 | 15.9 | 3.6 | 4.7 | 82 |
| 66 | $RuO_2$-BIPY[b] | 3[1-Octene] | 75.1 | 26.2 | 11.2 | 22.5 | 16.3 | 12.7 | 3.6 | 8.6 | 78 |

[a] Experimental series conditions; Ru, 3.0 mmole; $Bu_4PBr$, 5.0 g; 1-Octene, 200 mmole; 180°C; 83.7 Bars $CO/H_2$ (1:2).
[b] Experimental series conditions; Ru, 6.0 mmole; $Bu_4PBr$, 10.0 g; 1-Octene, 200 mmole; 180°C; 83.7 Bars $CO/H_2$ (1:2).

Example 67

Ruthenium(IV) oxide hydrate (1.146 g, 6.0 mmole) plus 2,2'-dipyridyl (0.937 g, 6.0 mmole) was dispersed in tetrabutylphosphonium bromide (20.0 g, 58.9 mmole), diluted with 1-decene (15.8 g, 100 mmole) and transferred in a glass liner under $N_2$ purge, to an 850 ml capacity pressure reactor equipped with heating and agitation means. The reactor was sealed, flushed with $CO/H_2$ and pressurized to 83.7 Bars with $CO/H_2$ (1:2). The mixture was heated to 180°C with rocking, held at this temperature for four hours and then allowed to cool.

Upon reaching ambient temperature, the reactor pressure (75.1 Bars) was noted, a typical gas sample taken, and the excess gas vented. The red liquid product (41.2 g) was analyzed by glc and Karl Fischer titration.

A typical liquid sample contained:

54.4 wt.% 1-undecanol
11.8 wt.% branched C-11 alcohols
3.8 wt.% C-11 aldehydes
1.5 wt.% water
9.6 wt.% decane
10.9 wt.% unreacted decenes.

Typical gas samples contained:

64 wt.% hydrogen
31 wt.% carbon monoxide
0.5 wt.% carbon dioxide.

Estimated linearity of the undecanol fraction is 82%. Estimated conversion of the undecene charge is 87%. Estimated yield of undecanols (basis decene charge) is 73 mole%.

Example 68 (Comparative)

Following the procedure of Example 26, the same 850 ml capacity pressure reactor is charged with a mixture of ruthenium(IV) oxide, (15.8 g, 100 mmole), 2,2'-dipyridyl (6.0 mmole) and 1-decene (15.8 g, 100 mmole). No tetrabutylphosphonium bromide fraction was added in this comparative example. The reactor was sealed, flushed with $CO/H_2$ and pressurized to 83.7 Bars with $CO/H_2$ (1:2). The mixture was heated to 180°C with rocking, held at that temperature for 4 hours and then allowed to cool.

The liquid product (18.2 g) contained:

23.8 % 1-undecanol
6.4% branched $C_{11}$ alcohols
0.1% $C_{11}$ aldehydes
0.3% water
10.9% decane
58.3% unreacted decenes.

Estimated linearity of the undecanol fraction is 79%. Estimated conversion of the decene charge is 41%. Estimated yield of undecanols product (basis decene charged) is 28 mole%.

It may be noted that in this comparative Example, both the conversion of olefins to oxo products, and the yield of desired undecanols, was significantly lower in the absence of the quaternary phosphonium salt, than in the previous Examples (e.g. Examples 26 and 67) where the salt is present throughout the oxonation.

Example 69 (Comparative)

Following the procedures of Example 67 and 68, the same 850 ml capacity pressure reactor was charged with a mixture of ruthenium(IV) oxide, hydrate (6.0 mmole) and 1-decene (15.8 g, 100 mmole). No tetrabutylphosphonium bromide or 2,2'-dipyridyl fractions were added in this comparative Example. The reactor was sealed, flushed with $CO/H_2$ and pressurized to 83.7 Bars with $CO/H_2$ (1:2). The mixture was heated to 180°C with rocking, held at that temperature for 4 hours and then allowed to cool.

The liquid product (19.8 g) contained:

36.7% 1-undecanol
23.9% branched $C_{11}$ alcohols
3.4% $C_{11}$ aldehydes
1.1% water
32.8% decane
0.2% unreacted decenes.

Estimated linearity of the undecanol fraction is 61%. Estimated yield of undecanols product (basis decene charged is 61 mole%).

It may be noted that in this comparative Example, both the yield of total undecanols and the linearity to the desired 1-undecanol, are significantly lower in the absence of the quaternary phosphonium salt and N-heterocyclic promoter, than in previous Examples (e.g. Example 67), where both the quaternary salt and promoter are present throughout the oxonation.

Example 70

A dispersion of ruthenium(IV) oxide, hydrate (6.0 mmole), plus 2,2'-dipyridyl (6.0 mmole) in tetrabutylphosphonium bromide (10.0 g, 29.5 mmole) was diluted with 11.2 g of 1-octene (100 mmole) and transferred in a glass liner under $N_2$ purge, to an 850 ml capacity pressure reactor as described in Example 26. The reactor was sealed, flushed with $CO/H_2$ and pressurized to 83.7 Bars with $CO/H_2$ (1:2). The mixture was heated to 180°C with rocking, held at that temperature for 4 hours and then allowed to cool.

The liquid product (25.2 g) contained:
68.7% 1-nonanol
8.6% 2-methyloctanol
1.7% 2-ethylheptanol
0.4% nonanals
2.0% water
9.1% octane
9.0% unreacted octenes.

Estimated linearity of the nonanol fraction is 87%. Estimated conversion of the octene charge is 89%. Estimated yield of nonanols product (basis octene charged) is 72 mole%.

Example 71 (Comparative)

The procedures of Example 70 were followed except that no tetrabutylphosphonium bromide was added.

The liquid product (14.1 g) contained:
37.4% 1-nonanol
7.9% 2-methyloctanol
0.2% 2-ethylheptanol
0.1% nonanals
4.0% water
11.7% octane
34.5% unreacted octenes.

Estimated linearity of the nonanol product fraction is 82%. Estimated conversion of the octene charge is 66%. Estimated yield of nonanols product (basis octene charged) is 38 mole%.

It may be noted that in this comparative Example, the octene conversion and yield of desired nonanol products was significantly lower in the absence of the tetrabutylphosphonium bromide than in Example 70 where said salt is present throughout the oxonation.

Example 72 (Comparative)

Example 70 was repeated, except that no 2,2'-dipyridyl fraction was employed.

The liquid product (25.3 g) contained:
50.7% 1-nonanol
24.6% 2-methyloctanol
3.1% 2-ethylheptanol
0.1% nonanals
0.5% water
12.3% octane
0.6% unreacted octenes.

Estimated linearity of the nonanol fraction is 65%. Estimated conversion of the octene charge is 99%. Estimated yield of nonanols product (basis octene charged) is 77 mole%.

It may be noted that in this comparative Example, the linearity to the desired 1-nonanol is significantly lower in the absence of the N-heterocyclic promoter, 2,2'-dipyridyl, than in Examples where this promoter is present throughout the oxonation.

Example 73 (Comparative)

The procedure of Example 70 was followed except that no quaternary phosphonium salt or N-heterocyclic promoter were employed.

The product (13.0 g) contained:
38.2% 1-nonanol
15.8% 2-methyloctanol
6.3% 2-ethylheptanol
0.4% nonanals
1.3% water
33.5% octane
1.8% octenes.

Estimated linearity of the nonanol fraction is 63%. Estimated yield of nonanols product (basis octene charged) is 50 mole%.

It may be noted that in this comparative Example, both the yield of desired nonanol products and the linearity to 1-nonanol was significantly lower in the absence of the quaternary phosphonium salt and N-heterocyclic promoter, than in Example 70, where both the said salt and said promoter are present throughout the oxonation.

Example 74

Ruthenium(IV) oxide hydrate (1.146 g, 6.0 mmole) plus 2,2'-dipyridyl (0.937 g, 6.0 mmole) was dispersed in tetrabutylphosphonium bromide (20.0 g, 58.9 mmole) and was diluted with 16.8 g of 1-dodecene (100 mmole) and transferred in a glass liner, under $N_2$ purge, to an 850 ml capacity pressure reactor. The reactor was sealed, flushed with $CO/H_2$ and pressurized to 83.7 Bars with $CO/H_2$ (1:2). The mixture was heated to 180°C with rocking, held at that temperature for 4 hours and then allowed to cool.

The liquid product (41.8 g) contained:
56.8% 1-tridecanol
13.4% branched $C_{13}$ alcohols
5.4% $C_{13}$ aldehydes
1.3% water
12.3% dodecane
9.9% unreacted dodecenes.

Estimated linearity of the tridecanol fraction is 81%. Estimated conversion of the dodecene charge is 88%. Estimated yield of tridecanols (basis dodecene charged) is 69 mole%.

Examples 75 to 85

In the Examples of Table 5, which follows, the same procedures were used as in previous Examples, except that a variety of tertiary amine promoters were used in conjunction with ruthenium(IV) oxide.

Generally, the most preferred amine promoters, basis the linearity and yields of desired alcohol product, are 2,2'-dipyridyl, 2,2'-dipyridyl amine (Example 81) and N,N,N',N'-tetramethylethylenediamine (Example 80).

In Examples 84 and 85, different ruthenium precursors were used in conjunction with said amine promoters.

TABLE 5[a]

| Example | Ruthenium catalyst | Promoter | Quaternary salt | Liquid product composition (%) Octenes | Octane | Nonyl aldehyde | Nonyl alcohols Branched | Nonyl alcohols Linear | Total nonanols yield (%) | Nonanol linearity (%) |
|---|---|---|---|---|---|---|---|---|---|---|
| 75 | $RuO_2xH_2O$ | DIPY[b] | $Bu_4PBr$ | 10.4 | 11.5 | 2.0 | 13.7 | 55.3 | 61 | 80 |
| 76 | " | 1,10-PHEN[b] | " | 4.2 | 11.4 | 2.0 | 18.9 | 55.7 | 74 | 75 |
| 77 | " | PY[b] | " | 9.8 | 0.1 | 0.2 | 24.7 | 40.8 | 64 | 62 |
| 78 | " | 3,5-LUT[b] | " | 12.2 | 0.1 | 0.3 | 25.0 | 47.3 | 69 | 65 |
| 79 | " | 2,6-LUT[b] | " | 0.4 | 17.4 | 0.5 | 26.3 | 40.3 | 64 | 61 |
| 80 | " | $Me_4EDA$[b] | " | 5.0 | 13.0 | 10.4 | 13.6 | 48.2 | 65 | 78 |
| 81 | " | DIPY-NH[b] | " | 9.7 | 8.4 | 19.3 | 7.1 | 42.5 | 56 | 84 |
| 82 | " | 2,3′-DIPY[b] | " | 0 | 16.4 | 1.8 | 22.1 | 47.9 | N.D.[c] | 68 |
| 83 | " | 2,4′-DIPY[b] | " | 0 | 22.4 | 0.2 | 38.6 | 38.5 | N.D.[c] | 57 |
| 84 | $Ru_3(CO)_{12}$ | DIPY | " | 9.6 | 11.3 | 2.9 | 7.5 | 44.9 | 47 | 86 |
| 85 | $Ru(AcAc)_3$ | DIPY | " | 9.3 | 12.0 | 0.4 | 16.8 | 43.5 | 56 | 72 |

[a] Reaction charge: Ru, 6.0 mmole; Ru/N ratio 1:2, $Bu_4PBr$, 10.0 g; 1-octene, 100 mmole.
Run conditions: 180°C; 83.7 Bars $CO/H_2$ (1:2) initial pressure; 4 hours.

[b] DIPY, 2,2′-dipyridyl; 1,10-PHEN, 1,10-phenanthroline; 3,5-LUT, 3,5-lutidine; 2,6-LUT, 2,6-lutidine; PY, Pyridine; $Me_4EDA$, N,N,N′,N′-tetramethylethylenediamine; DIPY-NH, 2,2′-dipyridylamine; 2,3′-DIPY, 2,3′-dipyridyl; 2,4′-DIPY, 2,4′-dipyridyl.

[c] N.D., not determined.

Example 86

Following the procedures of Example 26, the same 850 ml capacity pressure reactor was charged with a mixture of ruthenium oxide hydrate (1.146 g, 6.0 mmole) in conjunction with 2,2′-dipyridyl (0.937 g, 6.0 mmole), with a Ru/N ratio of 1:2. The mixture was dispersed in hexadecyltri-n-butylphosphonium bromide (10.0 g, 19.7 mmole) and diluted with 1-octene (22.4 g, 200 mmole). The reactor was sealed, flushed with $CO/H_2$ and pressurized to 83.7 Bars with $CO/H_2$ (1:2). The mixture was heated to 180°C with rocking, held at that temperature for 4 hours and then allowed to cool.

The liquid product (38.5 g) contained:

9.3 wt.% octenes
10.5 wt.% octane
1.7 wt.% 2-ethylheptanol
13.1 wt.% 2-methyloctanol
52.2 wt.% linear nonanol.

Estimated linearity of the nonanol fraction is 78%. Estimated yield of nonanols product (basis octene charged) is 61%.

Example 87

A dispersion of ruthenium(IV) oxide hydrate (6.0 mmole) plus 2,2′-dipyridyl (6.0 mmole) in tetrabutylphosphonium bromide (20.0 g, 58.9 mmole) was diluted with a mixed, internal $C_8$ olefin fraction (22.4 g, 200 mmole) and transferred in a glass liner, under $N_2$ purge, to the 850 ml capacity pressure reactor. The reactor was sealed, flushed with $CO/H_2$ and pressurized to 83.7 Bars with $CO/H_2$ (1:2). The mixture was heated to 180°C with rocking, held at that temperature for 4 hours and then allowed to cool.

Upon reaching ambient temperature, the reactor pressure (69.9 Bars) is noted, a typical gas sample taken, and the excess gas vented. The reddish-brown liquid product (47.1 g) contained:

26.4% 1-nonanol
14.4% 2-methyloctanol
4.2% 1-nonanal
1.9% 2-methyloctanal
1.0% water
43.0% unreacted octenes.

Typical gas samples contained:

55% hydrogen
38% carbon monoxide
4.0% carbon dioxide
3.2% methane.

Estimated linearity of the nonanol fraction is 65%. Estimated linearity of the nonanal fraction is 69%. Total $C_9$ alcohol+aldehyde yield (basis octene converted) is 79 mole%.

Example 88

A dispersion of ruthenium(IV) oxide hydrate (1.5 mmole) and 2,2′-dipyridyl (0.5 mmole) in tetrabutylphosphonium bromide (15.0 mmole) was diluted with a mixed $C_{11}$ internal olefin fraction (30.8 g, 200 mmole) and transferred in a glass liner under nitrogen purge, to a 300 ml capacity pressure reactor equipped with heating and agitation means. The reactor was sealed, flushed with $CO/H_2$ and pressurized to 83.7 Bars with $CO/H_2$ (1:1). The mixture was heated to 180°C with stirring, held at this temperature for 4 hours and then allowed to cool.

Upon reaching ambient temperature, the reaction pressure (63.7 Bars) is noted, a typical gas sample taken, and the excess gas removed. The red liquid product (34.0 g) contained:

12.1% n-undecane
7.2% undecenes
5.0% branched $C_{12}$ aldehydes
2.1% 1-dodecanal
25.1% branched $C_{12}$ alcohols
32.6% 1-dodecanol.

Typical gas samples contained:

51.1% hydrogen
32.6% carbon monoxide
9.4% carbon dioxide.

Example 89

A dispersion of triruthenium dodecacarbonyl (0.5 mmole) and 2,6-lutidine (1.5 mmole) in

tetrabutylphosphonium acetate (15.0 mmole) was diluted with $C_{11}$ internal olefin (30.5 g, 200 mmole) and transferred in a glass liner under nitrogen purge, to a 550 ml capacity pressure reactor equipped with heating and agitation means. The reactor was sealed, flushed with $CO/H_2$ and pressurized to 97.5 Bars with $CO/H_2$ (1:1). The mixture was heated to 180°C with rocking, held at this temperature for 18 hours and then allowed to cool.

Upon reaching ambient temperature, the reaction pressure (88.9 Bars) was noted, a typical gas sample taken, and the excess gas removed. The red liquid product (38.2 g) contained:

1.7% n-undecane
73.2% undecenes
1.3% branched $C_{12}$ aldehydes
1.5% 1-dodecanal
5.4% branched $C_{12}$ alcohols
6.3% 1-dodecanol.

Typical gas samples contained:

47.2% hydrogen
49.5% carbon monoxide
0.3% carbon dioxide.

The following Examples 90 to 110 illustrate the use of tertiary phosphine promoters.

Example 90

Ruthenium(IV) oxide hydrate (1.146 g, 6.0 mmole) and bis(1,3-diphenylphosphino)propane (2.475 g, 6.0 mmole) were dispersed in tetrabutylphosphonium bromide (10.0 g, 29.5 mmole), diluted with 1-octene (22.4 g, 200 mmole) and transferred in a glass liner, under $N_2$ purge, to the 850 ml capacity pressure reactor. The reactor was sealed, flushed with $CO/H_2$ and pressurized to 83.7 Bars with $CO/H_2$ (1:2). The mixture was heated to 180°C with rocking, held at this temperature for four hours and then allowed to cool.

Upon reaching ambient temperature, the reactor pressure (69.9 Bars) was noted, a typical gas sample taken, and the excess gas removed. The deep red liquid product (39.5 g) contained:

42.4 wt.% 1-nonanol
8.0 wt.% branched $C_9$ alcohols
1.0 wt.% $C_9$ aldehydes
1.2 wt.% water
17.4 wt.% n-octane
28.2 wt.% unreacted octenes.

Typical gas samples contained:

68 wt.% hydrogen
32 wt.% carbon monoxide
20 wt.% carbon dioxide.

Estimated linearity of the nonanol fraction is 84%. Estimated conversion of octene charge is 68%. Estimated total yield of nonanols (basis octene converted) is 67 mole%.

Example 91

Triruthenium dodecacarbonyl (1.78 g, 2.0 mmole) and bis(1,3-diphenylphosphino)propane (2.478 g, 6.0 mmole) were dispersed in tetrabutylphosphonium bromide (10.0 g, 29.5 mmole) and diluted with 1-octene (22.4 g, 200 mmole) and transferred in a glass liner, under $N_2$ purge, to the 850 ml capacity pressure reactor. The reactor was sealed, flushed with $CO/H_2$ and pressurized to 83.7 Bars with $CO/H_2$ (1:2). The mixture was heated to 180°C with rocking, held at this temperature for four hours and then allowed to cool.

Upon reaching ambient temperature, the reactor pressure (73.4 Bars) was noted, a typical gas sample taken, and the excess gas removed. The deep red liquid product (38.7 g) contained:

30.4 wt.% linear nonanols
8.4 wt.% branched nonanols
18.4 wt.% octenes
38.9 wt.% octane
1.3 wt.% nonanals.

Estimated linearity of the nonanol fraction is 78%. Estimated conversion of octene charge is 80%. Estimated total yield of nonanols basis octene converted is 42%.

Example 92

The technique of Examples 90 and 91 was followed, except that ruthenium acetylacetonate (2.390 g, 6.0 mmole) was the ruthenium compound, bis(1,3-diphenylphosphino)propane (2.475 g, 6.0 mmole) was the phosphine promoter, and the catalyst precursors were dispersed in $C_{16}H_{33}Bu_3PBr$ (10.0 g, 29.5 mmole).

After cooling the reactor pressure (83.7 Bars) was noted, a typical gas sample taken, and the excess gas removed. The deep red liquid product (41.6 g) contained:

28.6 wt.% linear nonanols
6.9 wt.% branched nonanols
5.3 wt.% octenes
46.1 wt.% octane
0.7 wt.% nonanals.

Estimated linearity of the nonanol fraction is 81%. Estimated conversion of octene charge is 94%. Estimated total yield of nonanols basis octene converted is 35%.

Examples 93 to 100

In the following Table 6, data are summarized for Examples 93 to 100, in which the ruthenium-containing compound is ruthenium(IV) oxide hydrate (6.0 mmole), the quaternary salt is tetra-n-butylphosphonium bromide (10.0 g, 29.5 mmole) and the olefin is 1-octene (22.4 g, 200 mmole). These runs were completed in the same manner as Examples 90 to 92, except that different di- and poly-phosphine promoters were used.

It may be noted that:

a) The $RuO_2$—$PH_2PCH_2CH_2CH_2PPh_2$ and $RuO_2$—$CH_3$—$C(CH_2PPh_2)_3$ catalyst precursors generated nonanol product with as high as 84% linearity.

b) The total yield of nonanol product in Example 93, with the $RuO_2$—$Ph_2PCH_2CH_2PPh_2$ combination, is 84 mole%.

c) The $RuO_2$—$Ph_2PCH_2PPh_2$ catalyst precursor in Example 99 gives nonanol product with 87% linearity.

TABLE 6[a]

| Example | Promoter | Liquid product composition (%) Unreacted octenes | Octane | Nonanals | Nonanols Branched | Nonanols Linear | Nonanol linearity |
|---|---|---|---|---|---|---|---|
| 93 | $Ph_2PCH_2CH_2PPh_2$ | 30.1 | 10.8 | 2.4 | 11.2 | 40.9 | 79 |
| 94 | $Ph_2PCH_2CH_2CH_2PPh_2$ | 28.2 | 17.4 | 1.0 | 8.0 | 42.4 | 84 |
| 95 | $(Ph_2PCH_2CH_2)_2$ | 19.0 | 10.5 | 6.9 | 19.3 | 38.0 | 66 |
| 96 | $CH_3—C(CH_2PPh_2)_3$ | 61.7 | 6.7 | 1.9 | 4.2 | 22.0 | 84 |
| 97 | $P(CH_2CH_2PPh_2)_3$ | 36.2 | 10.9 | 0.8 | 8.7 | 38.2 | 81 |
| 98 | $(Ph_2PC_5H_4)_2Fe$ | 26.8 | 6.1 | 8.6 | 8.0 | 29.4 | 79 |
| 99 | $Ph_2PCH_2PPh_2$ | 36.9 | 16.6 | 6.9 | 4.6 | 29.9 | 87 |
| 100 | $Ph_2P(CH_2)_5PPh_2$ | 13.9 | 9.8 | 6.6 | 21.6 | 38.1 | 64 |

[a] Reaction charge: Ru, 6.0 mmole; Ru: P (as phosphine)=1:2; $Bu_4PBr$, 29.5 mmole; 1-octene, 200 mmole.
Run conditions: 180°C, 83.7 Bars of $CO/H_2$ (1:2) initial pressure: 4 hours.

Examples 101 and 102 (Comparative)

The two Examples in Table 7 were conducted in the same manner in all respects as Examples 93 to 100, except the phosphine promoters used were monodentate phosphines. (e.g. $PPh_3$ and $PBu_3$). It may be noted that in these Examples, the nonyl alcohol linearity is Ca. 60%, considerably below the figures in Table 6 for ruthenium coupled with diphosphines, polyphosphines and phosphine substituted metallocenes. The data can be summarized as follows:

TABLE 7[a]

| | | Liquid product composition (%) | | | | | |
|---|---|---|---|---|---|---|---|
| Example | Promoter | Octenes | Octane | Nonanals | Branched | Linear | Linearity |
| 101 | $PPh_3$ | 1.9 | 16.2 | 0.1 | 30.4 | 45.2 | 60 |
| 102 | $PBu_3$ | 4.3 | 14.9 | 0.7 | 27.5 | 42.1 | 60 |

[a] Reaction charge: Ru, 6.0 mmole; Ru: $PR_3$, −1:2; $Bu_4PBr$, 29.5 mmole; 1-octene, 200 mmole. Run conditions: 180°C; 83.7 Bars of $CO/H_2$ (1:2) initial pressure; 4 hours.

Examples 103 to 108

The method used in Examples 103 to 108 was the same as that used in Example 90, except that mixed internal octenes were employed as the typical internal olefin feedstock. Table 8, which follows, records the preparation of predominantly linear nonyl alcohols from $CO/H_2$ plus mixed internal olefin fractions, particularly mixed $C_8$ olefin feedstocks, using ruthenium(IV) oxide, hydrate, in combination with certain diphosphine and polyphosphine promoters plus tetra-n-butylphosphonium bromide.

TABLE 8[a]

| Example | Ruthenium precursor | Promoter | Quaternary salt | Liquid product composition (%) Octenes | Octane | Nonanals | Nonanols Branched | Nonanols Linear | Proportion[b] of product |
|---|---|---|---|---|---|---|---|---|---|
| 103 | $RuO_2xH_2O$ | $Ph_2PCH_2CH_2PPh_2$ | $Bu_4PBr$ | 92.7 | 5.0 | 0.9 | 0.4 | 0.3 | 40 |
| | | | | [b] 50.1 | 2.5 | 4.5 | 14.7 | 16.2 | 60 |
| 104 | $RuO_2xH_2O$ | $Ph_2PCH_2CH_2CH_2PPh_2$ | $Bu_4PBr$ | 92.3 | 5.6 | 0.7 | 0.7 | 0.3 | 35 |
| | | | | [b] 49.5 | 2.7 | 3.2 | 21.2 | 16.5 | 65 |
| 105 | $RuO_2xH_2O$ | $(Ph_2PCH_2CH_2)_2$ | $Bu_4PBr$ | 87.9 | 6.2 | 3.6 | 0.8 | 0.6 | 16 |
| | | | | [b] 55.4 | 3.7 | 7.3 | 16.8 | 14.2 | 84 |
| 106 | $RuO_2xH_2O$ | $(Ph_2PC_5H_4)_2Fe$[c] | $Bu_4PBr$ | 95.5 | 2.2 | 0.3 | 0.3 | 0.1 | |
| | | | | [b] 51.3 | 3.2 | 1.8 | 14.2 | 23.8 | |
| 107 | $RuO_2xH_2O$ | $CH_3C(CH_2PPh_2)_3$[d] | $Bu_4PBr$ | 89.1 | 5.0 | 4.4 | 0.6 | 0.6 | 16 |
| | | | | [b] 48.1 | 2.9 | 10.6 | 13.3 | 16.4 | 84 |
| 108 | $RuO_2xH_2O$ | $P(CH_2CH_2CN)_3$[d] | $Bu_4PBr$ | 1.1 | 11.4 | 0.6 | 46.6 | 31.0 | |

[a] Reaction charge: Ru, 6.0 mmole; Ru:P, 1:2; $Bu_4PBr$, 58.9 mmole; mixed internal octenes, 200 mmole. Run conditions: 180°C; 83.7 Bars $CO/H_2$ (1:2) initial pressure, 4 hours.
[b] Two-phase liquid product.
[c] Reaction charge: Ru, 3.0 mmole; Ru:P, 1:2, $Bu_4PBr$, 29.5 mmole; mixed internal octenes, 100 mmole.
[d] Reaction charge: Ru, 1.5 mmole; Ru:P, 1.5:1; $Bu_4PBr$, 29.5 mmole; mixed internal octenes, 100 mmole.

Example 109

Ruthenium(IV) oxide hydrate (0.573 g, 3.0 mmole) and bis(2-diphenylphosphinoethyl)phenylphosphine (1.604 g, 3.0 mmole) were dispersed in tetrabutylphosphonium bromide (5.0 g, 14.7 mmole), diluted with mixed internal octenes (11.22 g, 100 mmole) and transferred in a glass liner, under $N_2$ purge, to the 850 ml capacity pressure reactor of Example 90, and the technique of that Example was repeated.

The two-phase liquid product composition (18.3 g) contained:

| | % Composition | |
|---|---|---|
| | 1st Phase | 2nd Phase |
| Octene | 91.9 wt.% | 43.4 wt.% |
| Octane | 2.8 wt.% | 1.7 wt.% |
| Linear nonanal | 1.0 wt.% | 4.7 wt.% |
| Branched nonanal | 0.3 wt.% | 0.7 wt.% |
| Linear nonanol | 1.9 wt.% | 37.5 wt.% |
| Branched nonanol | 0.1 wt.% | 2.2 wt.% |
| Water | 0.1 wt.% | 2.7 wt.% |

Estimated linearity of the nonanol fraction is 94%.

Example 110 (Comparative)

This Example shows the poorer performance for the ruthenium catalyst in the absence of a quaternary phosphonium salt and phosphine promoter.

A mixture of ruthenium(IV) oxide, hydrate (1.1469 g, 6.0 mmole) and 1-octene (22.4 g, 200 mmole) was reacted in the 850 ml capacity pressure reactor according to the technique of Example 90.

The two-phase liquid product (40.1 g, 45 ml) was analyzed by glc and Karl Fischer titration. The bottom phase (10 ml) proved to be water. The top phase (35 ml) contained:

13.0% 1-nonanol
7.8% branched $C_9$ alcohols
1.9% $C_9$ aldehydes
33.5% n-octane
6.5% unreacted octenes.

Estimated linearity of the nonanol fraction is 63%. Estimated conversion of octene charge is 91%. Estimated total yield of $C_9$ alcohols (basis octene converted) is 24 mole%.

In comparing these results with those of Example 90 it may be noted that:

a) The estimated total yield of $C_9$ alcohol product is higher in Example 90 (67 mole%), with added quaternary salt plus phosphine promoter than in comparative Example 110.

b) The production of by-product hydrocarbon, in this case octane, is lower in Example 90 than in the Example 110 (e.g. 17.4 wt.% octane versus 33.5 wt.%).

c) The linearity of the nonanol product fraction is higher in Example 90 (84%), versus 63% for Example 110.

**Claims**

1. A process for preparing alcohols and aldehydes by reacting $C_2$—$C_{30}$ terminal or internal olefins with carbon monoxide and hydrogen in the presence of a ruthenium catalyst at a temperature of at least 50°C and a pressure of at least 7.5 Bars characterized in that the ruthenium catalyst is dispersed in a low melting quaternary phosphonium or ammonium base or salt, or that the ruthenium catalyst, in association with a tertiary amine or phosphine promoter, is dispersed in a low melting phosphonium base or salt.

2. A process according to claim 1 characterized in that the temperature is from 100 to 220°C.

3. A proces according to claim 1 or 2 characterized in that the pressure is from 35 to 210 Bars.

4. A process according to any of claims 1 to 3 characterized in that the olefins are $C_8$—$C_{20}$ internal olefins.

5. A process according to any one of claims 1 to 4 characterized in that the quaternary phosphonium salt is a tetralkylphosphonium salt in which the alkyl groups each have 1 to 16 carbon atoms.

6. A process according to any one of claims 1 to 5 characterized in that the tertiary amine promoter is a bidentate N-heterocyclic compound.

7. A process according to claim 6 characterized in that the ruthenium catalyst comprises from $1 \times 10^{-5}$ to 30 weight percent ruthenium, the amine promoter comprises from $1 \times 10^{-5}$ to 30 weight percent, and the quaternary phosphonium salt comprises from 0.1 to 80 weight percent, based on the total weight of reaction mixture.

8. A process according to any one of claims 1 to 5 characterized in that the tertiary phosphine promoter is a bidentate or multidentate phosphine.

9. A process according to claim 8 characterized in that the ruthenium catalyst comprises from $1 \times 10^{-5}$ to 30 weight percent ruthenium, the phosphine promoter comprises from $1 \times 10^{-5}$ to 30 weight percent, and the quaternary phosphonium salt comprises from 0.1 to 80 weight percent, based on the total weight of reaction mixture.

**Patentansprüche**

1. Verfahren zur Herstellung von Alkoholen und Aldehyden durch Umsetzung von $C_2$ bis $C_{30}$-endständigen oder inneren Olefinen mit Kohlenmonoxid und Wasserstoff in Gegenwart eines Ruthenium-Katalysators bei einer Temperatur von mindestens 50°C und einem Druck von mindestens 7,5 bar, dadurch gekennzeichnet, daß der Ruthenium-Katalysator in einer niedrigschmelzenden quarternären Phosphonium- oder Ammonium-Base oder -Salz dispergiert wird oder daß der Ruthenium-Katalysator zusammen mit einem tert. Amin- oder Phosphin-Promotor in einer niedrig-schmelzenden Phosphonium-Base oder -Salz dispergiert wird.

2. Ein Verfahren nach Anspruch 1, dadurch gekennzeichnet, daß die Temperatur 100 bis 220°C beträgt.

3. Ein Verfahren nach Anspruch 1 oder 2, dadurch gekennzeichnet, daß der Druck 35 bis 210 bar beträgt.

4. Ein Verfahren nach einem der Ansprüche 1 bis 3, dadurch gekennzeichnet, daß die Olefine $C_8$ bis $C_{20}$-interne Olefine sind.

5. Ein Verfahren nach einem der Ansprüche 1 bis 4, dadurch gekennzeichnet, daß das quarternäre Phosphonium-Salz ein Tetraalkylphosphonium-Salz ist, in welchem die Alkylgruppen jeweils 1 bis 16 Kohlenstoffatome haben.

6. Ein Verfahren nach einem der Ansprüche 1 bis 5, dadurch gekennzeichnet, daß der tert. Amin-Promotor eine zweizähnige N-heterozyklische Verbindung ist.

7. Ein Verfahren nach Anspruch 6, dadurch gekennzeichnet, daß der Ruthenium-Katalysator aus $1 \times 10^{-5}$ bis 30 Gew.% Ruthenium besteht oder dieses enthält, $1 \times 10^{-5}$ bis 30 Gew.% Amin-Promotor und 0,1 bis 80 Gew.% quarternäres Phosphonium-Salz vorliegen, jeweils bezogen auf das Gesamtgewicht der Reaktionsmischung.

8. Ein Verfahren nach einem der Ansprüche 1 bis 5, dadurch gekennzeichnet, daß der tert. Phosphin-Promotor ein zweizähniges oder mehrzähniges Phosphin ist.

9. Ein Verfahren nach Anspruch 8, dadurch gekennzeichnet, daß der Ruthenium-Katalysator $1 \times 10^{-5}$ bis 30 Gew.% Ruthenium enthält oder daraus besteht, $1 \times 10^{-5}$ bis 30 Gew.% Phosphin-Promotor und 0,1 bis 80 Gew.% quarternäres Phosphonium-Salz vorliegen, jeweils bezogen auf das Gesamtgewicht der Reaktionsmischung.

**Revendications**

1. Procédé de préparation d'alcools et d'aldéhydes en faisant réagir des oléfines terminales ou internes en $C_2$—$C_{30}$ avec de l'oxyde de carbone et de l'hydrogène en présence d'un catalyseur au ruthénium, à une température d'au moins 50°C et sous une pression d'au moins 7,5 bars, caractérisé en ce que le catalyseur au ruthénium est dispersé dans une base ou un sel de phosphonium ou d'ammonium quaternaire à bas point de fusion, ou en ce que le catalyseur au ruthénium, associé à une amine tertiaire ou à une phosphine comme promoteur, est dispersé dans une base ou un sel de phosphonium à bas point de fusion.

2. Procédé suivant la revendication 1, caractérisé en ce que la température est de 100 à 220°C.

3. Procédé suivant les revendications 1 ou 2, caractérisé en ce que la pression est de 35 à 210 bars.

4. Procédé suivant l'une quelconque des revendications 1 à 3, caractérisé en ce que les oléfines sont des oléfines internes en $C_8$ à $C_{20}$.

5. Procédé suivant l'une quelconque des revendications 1 à 3, caractérisé en ce que le sel de phosphonium quaternaire est un sel de tétraalkylphosphonium dans lequel les groupes alkyle ont chacun 1 à 16 atomes de carbone.

6. Procédé suivant l'une quelconque des revendications 1 à 5, caractérisé en ce que l'amine tertiaire utilisée comme promoteur est un coposé N-hétérocyclique bicoordonné.

7. Procédé suivant la revendication 6, caractérisé en ce que la catalyseur au ruthénium comprend de $1.10^{-5}$ à 30% en poids de ruthénium, l'amine utilisée comme promoteur constitue de $1.10^{-5}$ à 30% en poids, et le sel de phosphonium quaternaire constitue de 0,1 à 80% en poids, par rapport au poids total du mélange réactionnel.

8. Procédé suivant l'une quelconque des revendications 1 à 5, caractérisé en ce que la phosphine tertiaire utilisée comme promoteur est une phosphine bicoordonnée ou multicoordonnée.

9. Procédé suivant la revendication 8, caractérisé en ce que le catalyseur au ruthénium comprend de $1.10^{-5}$ à 30% en poids de ruthénium, la phosphine utilisée comme promoteur constitue de $1.10^{-5}$ à 30% en poids, et le sel de phosphonium quaternaire constitue de 0,1 à 80% en poids, par rapport au poids total du mélange réactionnel.